(19) Europäisches Patentamt
European Patent Office
Office européen des brevets

(11) Publication number: 0 262 516 A2

(12) **EUROPEAN PATENT APPLICATION**

(21) Application number: 87113597.6

(51) Int. Cl.4: C12N 15/00

(22) Date of filing: 17.09.87

(30) Priority: 29.09.86 US 912950

(43) Date of publication of application: 06.04.88 Bulletin 88/14

(84) Designated Contracting States: CH DE FR GB LI NL

(71) Applicant: MILES LABORATORIES, INC.
1127 Myrtle Street
Elkhart Indiana 46514(US)

(72) Inventor: Nicholson, Mary Ann
329 Meisner Avenue
Elkhart, IN 46514(US)
Inventor: Sanders, Mary Ellen
52935 Marvin Street
Elkhart, IN 46514(US)

(74) Representative: Dänner, Klaus, Dr. et al
c/o Bayer AG Konzernverwaltung RP
Patentabteilung
D-5090 Leverkusen 1 Bayerwerk(DE)

(54) **Genetic transformation of lactic acid bacteria.**

(57) Non-protoplasted lactic acid-producing bacterial cells are genetically transformed with DNA by incubating a mixture of the cells with the DNA in a physiologically acceptable transformation solution containing a transformation-promoting amount of a water-soluble lower polyalkylene glycol.

EP 0 262 516 A2

Xerox Copy Centre

# GENETIC TRANSFORMATION OF LACTIC ACID BACTERIA

Non-protoplasted lactic acid-producing bacterial cells are genetically transformed with DNA by incubating a mixture of the cells with the DNA in a physiologically acceptable transformation solution containing a transformation-promoting amount of a water-soluble lower polyalkylene glycol.

## BACKGROUND OF THE INVENTION

### Field of the Invention

The present invention relates to genetic transformation of lactic acid-producing bacteria.

### Description of the Background Art

Lactic acid-producing bacteria are utilized extensively in the preparation of fermented milk products, such as cheeses, yogurts and the like, as well as in the fermentative production of numerous other food products. Examples of known lactic acid bacteria include species within the genera Streptococcus, Lactobacillus and Pediococcus.

There is an interest in applying recombinant DNA technology for improving lactic acid bacteria and for modifying their characteristics. However, it was previously thought that cell wall removal (protoplast formation) was essential for transformation of lactic acid bacteria, as has previously been reported for transformation of Streptococcus lactis. Kondo et al., Appl. Environ. Microbiol., 48:252-259 (1984).

Although transformation of protoplasted S. lactis cells with plasmid DNA has been accomplished, the poor understanding of protoplast formation and cell wall regeneration conditions, as well as DNA uptake mechanisms, has in practice caused difficulties in reproducing established protocols in different laboratories. Furthermore, transformation efficiencies of the known protoplast-dependent techniques are often disappointing. There thus remains a need for a method for genetically transforming lactic acid bacteria which does not possess the disadvantages of previously known techniques.

## SUMMARY OF THE INVENTION

In accordance with the present invention, DNA is mixed with gram-positive lactic acid-producing non-protoplasted bacterial cells in a physiologically acceptable aqueous solution containing at least about 5% by weight of a water-soluble, lower polyalkylene glycol. The mixture is incubated to genetically transform the cells with the DNA.

## BRIEF DESCRIPTION OF THE DRAWINGS

FIG. 1 graphically illustrates the effects of increasing amounts of DNA levels on number of transformants.

FIG. 2 graphically illustrates the effects of heat shock, time of incubation and recovery medium on transformation levels.

FIG. 3 graphically illustrates the effect of polyethylene glycol concentration on number of transformants.

FIG. 4 graphically illustrates the effect of pH of polyethylene glycol solution on transformation efficiency.

FIG. 5 graphically illustrates the effect of cell density on transformation.

FIG. 6 graphically illustrates the effect on transformation of acclimating cells in hypertonic solution prior to DNA addition.

## DESCRIPTION OF THE PREFERRED EMBODIMENTS

Applicants have surprisingly discovered a method for genetically transforming whole cells of lactic acid bacteria without the necessity for protoplasting the cells by enzymatically removing the bacterial cell walls.

The present invention is useful for genetically modifying and improving lactic acid bacteria, examples of which include species within the genera Streptococcus, Lactobacillus and Pediococcus .

Known species of lactic acid bacteria which have been recognized as being useful to the dairy industry include Streptococcus lactis, Streptococcus cremoris , Streptococcus diacetylactis, Streptococcus thermophillus, Streptococcusfaecium, Lactobacillus acidophilius, Lactobacillus alimentarius, Lactobacillus bifidus, Lactobacillus brevis Lactobacillus buchneri, Lactobacillus bulgaricus, Lactobacillus casei, Lactobacillus catenaforme, Lactobacillus cellobiosus, Lactobacillus collinoides, Lactobacillus confusus, Lactobacillus curvatus, Lactobacillus delbrueckii, Lactobacillus farciminis, Lactobacillus fermentatae, Lactobacillus fermentum, Lactobacillus fructivorans, Lactobacillus fructosus, Lactobacillus helveticus, Lactobacillus heterohiochi, Lactobacillus hilgardii, Lactobacillus homohiochi, Lactobacillus jensenii, Lactobacillus lactis, Lactobacillusleichmannii, Lactobacillus malefermentans, Lactobacillus mali, Lactobacillus maltaromicus, Lactobacillus minutus, Lactobacillus pentoaceticus, Lactobacillus plantarum, Lactobacillus rogosae, Lactobacillus ruminis, Lactobacillus sake Lactobacillus lalivarius, Lactobacillus sanfrancisco, Lactobacillus thermophilus, Lactobacillus trichodes , Lactobacillus viridescens, Lactobacillus vitulinus, and Lactobacillusxylosus.

Other lactic acid bacteria which have heretofore been recognized as being useful in the fermentative production of food products include: Pediococcus halophilus, Pediococcus acidilactici, Tetracoccus soyae, Streptococcus faecium, Streptococcus faecalis, Lactobacillus delbrueckii, and Lactobacillus casei .

The invention will be further described with reference to genetic transformation of Streptococcus lactis strain LM0230, but it is to be understood that the invention may also be employed for the genetic modification of other lactic acid bacteria, such as those referred to in the preceding paragraph.

Prior to transformation, S. lactis cells can be grown in a nutrient fermentation medium containing assimilable sources of carbon and nitrogen, as well as essential trace elements and growth factors, to an optical density (O.D.) at 600 nm of, for example, 0.89 (or about $10^8$ cells/ml). The cultivated cells then can be harvested from the broth, e.g., by centrifugation, and washed.

The washed, non-protoplasted cells are suspended in a physiologically acceptable solution for transformation.

In a particularly preferred embodiment, the cells are suspended in a physiologically acceptable hypertonic solution (such as 0.5 M sucrose or 0.5 M sorbitol solution, buffered with 0.01 M Tris, pH 7.0) to acclimate the cells for transformation. Incubation at 37°C for about one hour has been found to acclimate the cells for transformation.

Incubating the cells in the above-described solution to acclimate the cells for transformation has been found to substantially improve the transformation efficiency of non-protoplasted cells. For example, incubating non-protoplasted cells in 0.5 M sucrose at 37° for about one hour has resulted in about a 2 log cycle increase in transformant recovery compared to incubating the cells in 0.5 M sucrose solution at 37° for 15 minutes.

The acclimated cells can be pelleted by centrifugation and mixed with naked DNA (either linear or circular ized plasmids) in a physiologically acceptable hypertonic solution for transformation of the cells with DNA. It has been found that levels of transformants increase with increased DNA concentrations over a range of 0.48 µg DNA to 11.9 µg DNA per 0.5 ml aliquot of non-protoplasted cells suspended in sucrose solution. Over this range and under the conditions used, transformation efficiency was highest at the lowest level of DNA tested (0.48 µg). This phenomenon permits a balancing between the conservation of DNA and the maximization of the number of transformants.

With regard to cell density, however, the method of the present invention has been found to provide the greatest transformation efficiency at the highest cell densities tested (optical density (O.D.) at 600 nm of 0.89 or a colony count of $1 \times 10^8$ cfu/ml). This contrasts with previously known protoplast-dependent transformation procedures where low cell densities are recommended. Transformants were recovered from cell densities of O.D. 0.13 to 0.89.

To form a transformation mixture, the solution containing non-protoplasted cells and DNA is combined with a solution containing a transformation-promoting amount of a water-soluble lower polyalkylene glycol, e.g., about 5% by weight or more water-soluble polyethylene glycol (PEG) or low molecular weight polypropylene glycol (PPG).

One embodiment of the invention utilizes PEG solution containing about 30-35 weight percent polyethylene glycol having an average molecular weight within the range of from about 1,000 to about 8,000.

In a preferred embodiment, the polyalkylene glycol (e.g., PEG) solution is at about 0°C and has a volume of 3 to 4 times that of the sucrose solution containing the DNA/cell mixture with which it is combined.

In a more preferred embodiment, a polyethylene glycol solution is prepared by dissolving polyethylene glycol having an average molecular weight of about 3000 or higher in a buffered, aqueous solution containing about 0.5 M sodium succinate.

In a particularly preferred embodiment, the average molecular weight of the polyethylene glycol is about 5000 or higher.

As noted above, the polyethylene glycol solution advantageously is at about 0°C when combined with the sucrose solution containing non-protoplasted cells and DNA, although transformation has been achieved with polyethylene glycol solutions at room temperature. In preferred embodiments, about a 35 weight percent solution of 6,000-8,000 molecular weight polyethylene glycol at about 0°C is utilized.

Polyethylene glycol has been found effective in promoting transformation over a wide range of pH values, e.g., within the range of from about pH 5.0 to about pH 8.5. In a particularly preferred embodiment, the pH of the transformation solution is about 6.5-7.0.

Combining the 30-35 weight percent polyethylene glycol solution with the hypertonic solution containing non-protoplasted cells and DNA provides a transformation mixture in a solution comprised of from about 20-35% by weight polyethylene glycol. Most preferably, the solution containing the transformation mixture contains from about 25% to about 30% by weight polyethylene glycol.

In preferred embodiments, the hypertonic transformation solution, containing non-protoplasted cells, DNA and polyethylene glycol, is incubated to effect transformation of the non-protoplasted cells with the DNA. According to one embodiment, the transformation mixture is incubated at about room temperature for about 20 minutes and thereafter heat-shocked at a transformation-promoting temperature for a transformation-promoting period of time, for example, at a temperature of from about 40°C to about 45°C for from about 2 to about 10 minutes.

In a particularly preferred embodiment, the transformation mixture is heat-shocked at about 42°C for about 5 minutes after about 20 minutes incubation at room temperature. A heat shock at 42°C for 5 minutes following incubation of the transformation solution mixture has been found to result in a two-fold recovery of transformants over elimination of the heat shock step.

Transformed cells are amplified by growing them on a nutrient selection medium, such as agar plates. Advantageously, the plating medium contains high levels of non-nutritive carbohydrate. In a particularly preferred embodiment, the transformed cells are cultivated on an osmotically stable nutrient plating medium, such as nutrient agar containing about 0.5 M sucrose as an osmotic stabilizer. Plating the transformed cells onto an osmotically stabilized medium has been found to facilitate cell transformant recovery. Without being bound to any particular theory, it is possible that there is some degree of osmotic sensitivity of the cells after transformation.

For selecting transformants, the DNA utilized to transform the non-protoplasted cells advantageously includes one or more selectable phenotypic markers. Selection of transformants based on phenotype is well known. A common example of a selectable phenotypic marker is a gene coding for antibiotic resistance. Transformants then can be selected by plating the cells on a medium which inhibits growth of non-transformed cells, such as an agar containing antibiotic to which cells have been made resistant by a gene carried on the inserted DNA.

The invention is further illustrated by the following examples, which are not intended to be limiting.

## EXAMPLE I

Whole cell transformants in S. lactis using pSA3 and c2ϕ DNA

S. lactis strain LM0230 (originally designated $C14_5$, described in Klaenhammer et al., Appl. Environ. Microbiol., 35:592-600 (1978)) was inoculated from a frozen seed vial into 10 ml of M17-glucose broth (Terzaghi et al., Appl. Environ. Microbiol., 29:807-813 (1975)) and grown overnight at 32°C. M17-glucose broth (sometimes called M17-glu) is a modified Terzaghi et al. broth with glucose substituted for lactose, and includes 2.5 g yeast extract, 5.0 g phytone peptone, 5.0 g polypeptone peptone, 5.0 g beef extract, 5.0 g glucose, 19 g β-glycerophosphate, 0.5 g ascorbic acid, 1 ml 1 M $MgSO_4$ and 1 L $H_2O$.

A 0.3 ml sample of the cells grown overnight was transferred into 30 ml of M17-glucose broth and grown in 32°C water bath for 2 hours. The cells then were harvested by centrifugation at 6,000 rpm (SS34 sorval rotor) for 15 minutes. The harvested cells were washed once with 20 ml ice cold 0.01 M Tris, pH 7.0 (prepared by dissolving 1.21 g Tris base in 1 L deionized $H_2O$ and adjusting to pH 7.0 with HCl followed by sterilization in an autoclave), and pelleted again at 6,000 rpm.

The pelleted cells were resuspended in 7.6 ml of 0.5 M sucrose (in 0.01 M Tris, pH 7.0), and incubated at 37°C for 60 minutes. The cells were again pelleted, but at 5,000 rpm for 10 minutes at 15°C. The cells then were resuspended in 1 ml of "SMMB" solution prepared as follows: mix 85.58 g sucrose, 1.16 g maleic acid, 2.03 g $MgCl_2 \bullet 6H_2O$ and 350 ml $dH_2O$, adjust pH to 7.0, add 5.0 g BSA and bring to 500 ml with $H_2O$. This suspension was divided into 0.5 ml aliquots in clean, sterile tubes, to which was added 4 µg DNA (pSA3 or c2$\phi$) mixed 1:1 with $2^{\times}$ "SMM" solution prepared as follows: mix 85.58 g sucrose, 1.16 g maleic acid, 2.03 g $MgCl_2 \bullet 6H_2O$ and 150 ml $dH_2O$, adjust pH to 6.5 and bring to 250 ml with $H_2O$ (the DNA had previously been purified through CsCl-ethidium bromide centrifugation and dialyzed to remove salt in 10 mM Tris, 1 mM EDTA, pH 8.0).

To each of the 0.5 ml aliquots containing pSA3 or c2$\phi$ DNA was added 1.8 ml of 35% 8,000 molecular weight polyethylene glycol solution (PEG 8000) thawed on ice, to form a transformation mixture. The polyethylene glycol (PEG 8000) solution was prepared by a modified procedure of Wirth et al., J. Bacteriol., 165:831-836 (1986), with sodium succinate substituted for sucrose (100 mM Tris, 40 mM $CaCl_2$, 10 mM $MgCl_2$, 500 mM sodium succinate). The transformation mixture was allowed to incubate at room temperature for 20 minutes, then heat shocked 5 minutes at 42°C.

The transformation solution then was diluted with 5 ml of SMMB and spun at 5,000 rpm for 10 minutes. The cells were washed again with 5 ml of SMMB, and spun at 5,000 rpm for 10 minutes. The transformed cells then were resuspended in 1 ml ($2^{\times}$ SMM):(M17-glu) and transformation was induced with 0.05 µg/ml erythromycin. Thirty minutes were allowed for expression at room temperature and the cells were plated by adding 12 ml of tempered SM17 soft agar directly into the tubes and plated 3 ml/plate. (SM17 agar is prepared as described above for M17 glucose broth, to which additionally is added 71.1 g sucrose and agar. Plates were prepared as follows: overlay, 7 g/l; bottom agar, 15 g/l). The plates were incubated after inoculation for 2-4 days at 32°C. Plasmid pSA3 transformants were selected using 10 µg/ml erythromycin while c2$\phi$ transfectants were selected as plaque-forming units in LM0230 indicator cells (c2$\phi$ described in Jarvis et al., Appl. Environ. Microbiol., 51:1212-1277 (1986)).

EXAMPLE II

Transfection of protoplasted and non-protoplasted cells of S. lactis strain LM0230 with c2φ DNA

Transfection of non-protoplasted cells and cells protoplasted by varying enzyme treatments was compared, the conditions and results of which are shown in Table 1 below.

TABLE 1

c2 φ DNA entry with and without enzyme treatment of LM0230

| Enzyme Used | Transfectants/ml | | | |
|---|---|---|---|---|
| | + Enzyme | | - Enzyme | |
| | + DNA | - DNA | + DNA | - DNA |
| Mutanolysin[1] | 300 | <10 | <10 | <10 |
| Novozyme[2] | 70 | <10 | 350 | <10 |

1 Method used was according to Kondo et al., *supra*, with the following modifications: LM0230 indicator cells were plated with transfectants on M17-glu for plaque detection; mutanolysin was used at a final concentration of 6.25 μg/ml.

2 Method used was according to Example I with the following modifications: Novozyme (0.66 mg/ml, final concentration) was added prior to one hour incubation at 37°C; heat shock was omitted; PEG-1000 was used instead of PEG-8000; indicator cells were plated with transfectants on M17-glu agar.

The data in Table 1 indicates that the highest level of transfectants was observed without enzyme using essentially the method of Example I. Note that the procedure published by Kondo et al. (*supra*) modified by omission of mutanolysin treatment resulted in detection of no transfectants.

EXAMPLE III

Temperature effect on transformation with polyethylene glycol treatment

In this example, the procedures set forth in Example I were used with modifications indicated in Table 2 below.

TABLE 2

Transformation[1] of pSA3 into LM0230 using 30% PEG and 35% PEG at room temperature (RT) and 0°C

| PEG temp | Colonies | | | |
|---|---|---|---|---|
| | 30% PEG | | 35% PEG | |
| | Total | per µg[2] | Total | per µg[1] |
| RT | 63 | 5.3 | 267 | 22.4 |
| 0°C | 52 | 4.4 | 625 | 52.5 |

1 Method used is as in Example I with the following modifications: No heat shock was used; PEG-1000 replaced PEG-8000.

2 11.9 µg DNA was used per experiment.

Table 2 shows that a polyethylene glycol (PEG) temperature of 0°C produced favorable results using 35% PEG.

EXAMPLE IV

Effect of polyethylene glycol treatment on S. lactis strain LM-0230 using c2ϕ DNA

The effect of polyethylene glycol on transfection is shown in Table 3. When PEG was omitted from the procedure, no transfectants were isolated, indicating that PEG was required for transfection. Furthermore, the pattern found in Table 2 for pSA3 DNA was repeated in Table 3 for c2ϕ DNA, i.e, that maximum DNA uptake was seen in reactions using 35% PEG near 0°C.

TABLE 3

Transfectant[1] of LM0230 with c2 ϕ DNA using 30 and 35% PEG at room temperature (RT) and 0°C

| PEG | | + DNA | | - DNA |
|---|---|---|---|---|
| | | Total | per μg[3] | Total |
| 30% | RT | 320 | 52 | ND[2] |
| | 0°C | 1000 | 100 | <10 |
| 35% | RT | 880 | 140 | ND |
| | 0°C | 1600 | 260 | <10 |
| NO PEG | | <10 | <0.16 | <10 |

1 Procedure used is as in Example I with modifications stated in Table 2.

2 ND - not determined.

3 6.2 μg DNA used per experiment.

EXAMPLE V

Effect of DNA concentration on transformation

Strain LM0230 transformants were produced according to the method of Example I except that DNA concentrations ranged from 0 to 11.9 µg DNA added per transformation reaction, heat shock was omitted and PEG-1000 was used instead of PEG-8000. Figure 1 illustrates that increased DNA input results in increased recovery of transformants for all DNA concentrations examined, but that the highest transformation efficiency per microgram DNA was observed at the lowest DNA concentration tested (0.4 µg/DNA).

EXAMPLE VI

Effect of polyethylene glycol molecular weight on transformation efficiency of S. lactis strain LM0230 with plasmid pSA3 DNA

To determine the effect of PEG molecular weight on transformation efficiency, transformation with pSA3 DNA using PEG with average molecular weights of 1000, 3350 and 8000 was conducted according to the procedures set forth in Example I as modified according to Table 4 below.

TABLE 4

Effect of molecular weight of PEG on transformation[1] efficiency of pSA3 DNA into LM0230

| MW of PEG (35%) | Transformants/ml + DNA | Transformants/ml -DNA |
|---|---|---|
| 1000 | $4.9 \times 10^2$ | <1 |
| 3350 | $1.5 \times 10^3$ | <1 |
| 8000 | $2.4 \times 10^3$ | <1 |
| 3350 (sucrose)[2] | $2.6 \times 10^2$ | <1 |

1 Transformation was conducted as indicated in Example I with the following modifications: 35% PEG (at MW shown above), pH 7.5, was used; cells were grown 2 hrs. prior to harvesting. 2.2 µg DNA used/experiment.

2 Sucrose substituted for sodium succinate when preparing PEG solution.

As shown in Table 4, the highest level of transformants was seen with PEG-8000. Sucrose substituted for sodium succinate yielded about 1 log cycle fewer transformants.

EXAMPLE VII

Effect of heat shock treatment and osmotically stabilized plating media on transformant recovery

In this example, parameters affecting recovery of transformants were examined including the utility of the heat shock step set forth in Example I and a comparison using plating media with or without 0.5 M sucrose as an osmotic stabilizer. The results are shown in Fig. 2, which shows the positive effect of a heat-shock treatment (42°C/5 min.) on transformant recovery. Levels of transformants doubled with this treatment.

EXAMPLE VIII

Whole cell transformation of S. lactis strain LM0230 using various plasmids

The transformation procedures set forth in Example I were followed in every essential detail, except that LM0230 cells inoculated and grown overnight were propagated at 32°C for 4 hours ($OD_{600}$ = 0.9), and 30% PEG-8000 (pH 6.5) was substituted for 35% PEG-8000.

Plasmids used for transformations are listed in Table 7 below.

TABLE 7

Plasmid descriptions

| Plasmid | Mol. Wt. kb | Markers[1] |
|---|---|---|
| pSA3[2] | 10.2 | $Em^r$ |
| pVA856 | 9.1 | $Em^r$ |
| pAN50 | 13.2 | $Em^r$, $Cm^r$ |
| pTV1 | 12.4 | $Em^r$, $Cm^r$ |
| pME1a | 16.0 | Phage resistance |

1 Expressed in Streptococci. $Cm^r$ - chloramphenicol resistance. $Em^r$ - erythromycin resistance.

2 Dao, M.L. and Ferretti, J.J., Appl. Environ. Microbiol., 49, 115-119 (1985)

DNA Isolation

Plasmids pSA3, pAN50 and pVAP56 were isolated from Escherichia coli strains according to Silhavy, et al., Experiments with Gene Fusions, pp. 147-148 (1984). Small scale DNA isolations from 5 ml culture were prepared for quick plasmid screening. For DNA used in transformations, the procedure was scaled up for 1 L cells. Isopropanol precipitated DNA was loaded into a 40 ml cesium chloride-ethidium bromide gradient, refractive index 1.3970, and spun in a Beckman Ti60 rotor at 47,000 rpm for 48 hours. DNA was dialyzed overnight in TE buffer (10 mM Tris, 1 mM EDTA, pH 8.0), and precipitated with 2 volumes 95% ethanol and 0.1 volume 3 M sodium acetate (pH 5.2). DNA was resuspended in TE buffer to indicated concentrations. Chromosomal DNA was isolated from LM0230 transformants using a modification of the Marmur, J. Mol. Biol., 3:208-218 (1961) method. A 10% inoculum from overnight M17-glu plus antibiotic culture was made into 9 ml M17-glu broth + 20 mM D, L-threonine, and cultures were incubated 2 hrs./32°C. Cells were harvested, resuspended in 1 ml cold 0.01 M Tris, pH 7.0, and transferred to 1.5 ml Eppendorf tube. Cells were pelleted and resuspended in 500 µl 25% sucrose, 5.0 mM Tris, pH 8.0, 2 mM EDTA. To these cells, 6 µl ribonuclease A (10 mg/ml), 70 µl lysozyme (15 mg/ml in water) were added and suspension was held 1 hour at 37°C. 4 µl of 10% SDS was added. Suspension was inverted to mix and held at room temperature for 20 minutes. DNA was fragmented by vortexing 20 seconds. Two 50 mM Tris, pH 8.0-saturated phenol:chloroform (1:1) extractions were followed by three chloroform:isoamyl alcohol (24:1) extractions. DNA was passed through a membrane centricon filter (Amicon, Danvers, MA) and concentrated to 100 µl.

Plasmid DNA was isolated from S. lactis using the procedure of Anderson and McKay, Appl. Environ. Microbiol., 46:549-552 (1983), except in the case of pME1a DNA preparation. Plasmid pME1a was purified from LM0230 using the lysis and CsCl banding procedures of Klaenhammer et al., Appl. Environ. Microbiol., 35:592-600 (1978).

Transformation

Variations from the procedure of Example I not noted above are noted below. The compositions of buffers used were as follows. Buffer solutions SMM (0.5 M sucrose, 0.02 M malaete, 0.02 M $MgCl_2$, pH 6.5) and SMMB (SMM with 5% bovine serum albumin) are previously known and have previously been used, for example, as described by Kondo et al. (supra) and by Kondo and McKay, J. Dairy Sci., 65:1428-1431 (1982). Polyethylene glycol solutions were made according to the modified Wirth et al. procedure noted above in TSCM (100 mM Tris, 40 mM $CaCl_2$, 10 mM $MgCl_2$, 500 mM sodium succinate) at indicated pH. Sugar and salt solutions were autoclaved separately, and thereafter combined. PEG solutions made in TSCM were filtered through 0.45 μ filter, divided in 2 ml aliquots and stored -20°C until use. Transformants were recovered on M17-glu agar supplemented with 0.5 M sucrose (SM17-glu) bottom and overlay agars contained 1.5% and 0.5% agar, respectively.

Fig. 3 shows the effect of PEG-8000 prepared in TSCM and used at concentrations from 0 to 35%. These results show that 30% PEG-8000 gave maximum transformant yields, with essentially no transformation in the absence of PEG.

The effect of PEG solution pH was also tested. The data in Fig. 4 show that PEG is effective over a wide range of pH values (5.5 to 8.3). However, a pH of 4.4 effectively prevented transformation.

To determine the effect of cell density on transformation efficiency, cells of LM0230 were grown at 1% inoculum for 1.5, 2.0, 3.0, and 4.0 hr. at 32°C. Transformants were recovered at greater efficiencies at the highest cell densities tested (Fig. 5). The 4.0 hr. cells corresponded to an $A_{600}$ of 0.88 with viable cells of 1 $\times$ $10^8$/ml. This contrasts with protoplast procedures where low cell densities are recommended (Kondo et al. 1984, supra).

Cell incubation in 0.5 M sucrose prior to DNA addition was tested. It was found that shortening the holding time in sucrose at 37°C from 60 min. to 15 min. resulted in about a 2 log cycle drop in transformant recovery (Fig. 6). Therefore, this step appears to be important in cell adaptation to transformability.

In addition, the ability to use frozen cells of LM0230 was tested. Cells grown to normal levels were pelleted and frozen overnight at -70°C as pellets (no stabilizer) or in 0.5 M sucrose, 0.01 M Tris (pH 7.0). Cells were thawed and transformed with 0.5 μg pSA3 DNA along with freshly grown cells as a control. Both frozen cell preparations transformed equivalently well, at levels approximately 1 log cycle lower than control counts. The transformability of pSA3 isolated from 10 ml LM0230 cells using the procedure of Anderson and McKay (supra) without purification of the plasmid through a CsCl-EB gradient was determined. 7.1 $\times$ $10^3$ transformants/ml were obtained from plasmid DNA from 10 ml cells. Therefore, purification of DNA through density gradients is not required for transformation, although transformation efficiency is decreased.

The ability to apply this transformation system to linear DNA from ligation reaction mixes enables direct cloning of genes into LM0230 without prior selection into E. coli. The present transformation system was proven to be suitable for DNA in this form by testing with pSA3 (0.6 μg) DNA linearized with EcoRV mixed with pME1a (12.4 μg) DNA digested with EcoRV. These digests were incubated overnight with 400 units T4 DNA ligase. As controls, CsCl purified pSA3 (0.5 μg) and pSA3 religated to itself (2.4 μg) were tested. DNA was transformed into LM0230 with selection for $Em^r$. The results are shown in Table 8 below.

TABLE 8

The use of whole-cell transformation of LM0230 for direct cloning of pME1a onto pSA3

| Cloning Reaction | Amt. (μg) pSA3 used | Transformants / μg | Transformants / ml |
|---|---|---|---|
| pSA3 (CsCl) | 0.5 | $5.2 \times 10^5$ | $2.6 \times 10^5$ |
| pSA3 - religated | 2.4 | $3.3 \times 10^3$ | $7.8 \times 10^3$ |
| pSA3 + pME1a | 0.6 | $5.8 \times 10^3$ | $3.5 \times 10^3$ |

12.4 μg pME1a was ligated to pSA3.

Table 8 shows that linear and/or religated DNA transformed at approximately 2 log cycle lower efficiency. However, the levels of transformants obtained were suitable for establishing a partial gene bank of pME1a into pSA3. Twenty-one transformants selected at random were assayed for plasmid content. Of these, 6 showed insert DNA (Fig. 6), establishing the utility of this transformation system for direct cloning.

Additional plasmids were tested for transformability into LM0230, including pVA856, pAN50 and pTV1. Although the frequencies of transformation of these plamids was somewhat lower, in each case transformants were isolated. The presence of plasmid DNA was confirmed by agarose gel electrophoresis. When $Em^r$transformants of pTV1, a Tn917 delivery vehicle, were tested for plasmid DNA, none was apparent. Transformants were selected based on $Em^r$, a marker maintained on Tn917. Therefore, if transformation was followed by transposition, the $Em^r$ phenotype could result from Tn917 carried on the chromosome. This was tested by preparing chromosomal DNA from four of transformants, cutting with EcoRI and probing with $^{32}P$-labeled pTV1. The results indicated that pTV1 DNA was present in chromosomal DNA preparations of the transformants, since the hybridizing EcoRI fragments were of different sizes from each other and from pTV1. Whether the integration of DNA from pTV1 was due to transposition or homologous recombination is not known. However, all transformants were $Em^r$ but $Cm^s$, suggesting that Tn917 was integrated without the retaining pTV1 plasmid DNA.

The present invention provides a transformation procedure for the lactic acid bacteria which is not protoplast-dependent, yet is more consistent and reliable than prior art protoplast-dependent methods. Furthermore, transformants have been obtained at levels of $5 \times 10^5$ transformants/µg DNA, higher than any known transformation frequence reported for the lactic acid bacteria.

## Claims

1. A method for genetically transforming gram-positive lactic acid-producing bacteria with DNA, which comprises:

(a) forming a mixture of DNA with gram-positive, lactic acid-producing, non-protoplasted bacterial cells in a physiologically acceptable transformation solution containing at least about 5% by weight water-soluble lower polyalkylene glycol; and

(b) incubating the mixture to genetically transform the cells with the DNA.

2. The method of claim 1 wherein said transformation solution is a hypertonic solution.

3. The method of any of claims 1 and 2 wherein prior to forming said mixture, said cells are incubated in a physiologically acceptable hypertonic solution to acclimate the cells for transformation.

4. The method of any of claims 1 to 3 wherein said lower polyalkylene glycol is polyethylene glycol or polypropylene glycol.

5. The method of any of claims 1 to 4 wherein the mixture containing solution comprises from about 20 % to about 35 % by weight said polyethylene glycol.

6. The method of any of claims 1 to 5 wherein the mixture is incubated at about room temperature to transform the cells and wherein the mixture is heat shocked following the incubation step.

7. The method of any of claims 1 to 6 wherein the mixture containing solution is at a pH within the range of about 5.0-8.5.

8. The method of any of claims 1 to 7 further including the step of cultivating the transformed cells on an osmotically stable nutrient plating medium.

9. The method of any of claims 1 to 8 wherein said bacterial cells are of the genus Streptococcus, Lactobacillus or Pediococcus.

10. A method for genetically transforming Streptococcus lactis with naked DNA comprising:

(a) incubating non-protoplasted Streptococcus lactis cells in a hypertonic solution which otherwise is physiologically acceptable to the cells, to acclimate the cells for transformation;

(b) forming a first mixture of the acclimated non-protoplasted cells and naked DNA in a solution which is hypertonic with respect to the cells, but otherwise physiologically acceptable to said cells;

(c) combining with said first mixture a polyethylene glycol solution to form a transformation mixture, the polyethylene glycol solution being at about 0°C and having about 3 to 4 times the volume of the first mixture, and containing about 30-35 weight percent polyethylene glycol having an average molecular weight of 5,000 or higher;

(d) incubating the transformation mixture at about room temperature for about 20 minutes;

(e) heat-shocking the incubated transformation mixture for from about 2 to about 10 minutes at about 40-45°C; and

(f) cultivating the transformed cells on an osmotically stable nutrient plating medium.

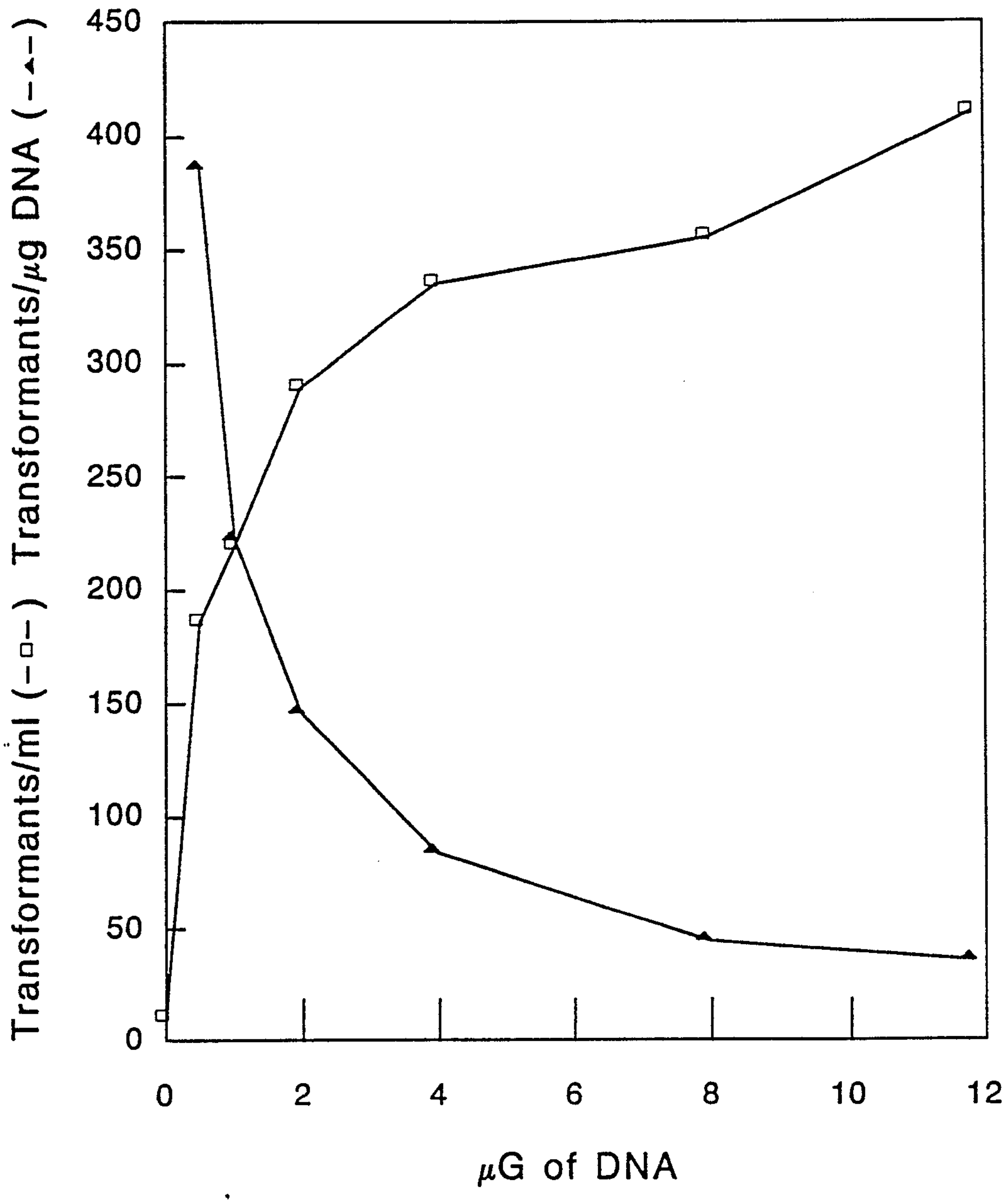
Transformants/ml (-□-) Transformants/μg DNA (-▲-)
450
400
350
300
250
200
150
100
50
0
0
2
4
6
8
10
12
μG of DNA

FIG. 1

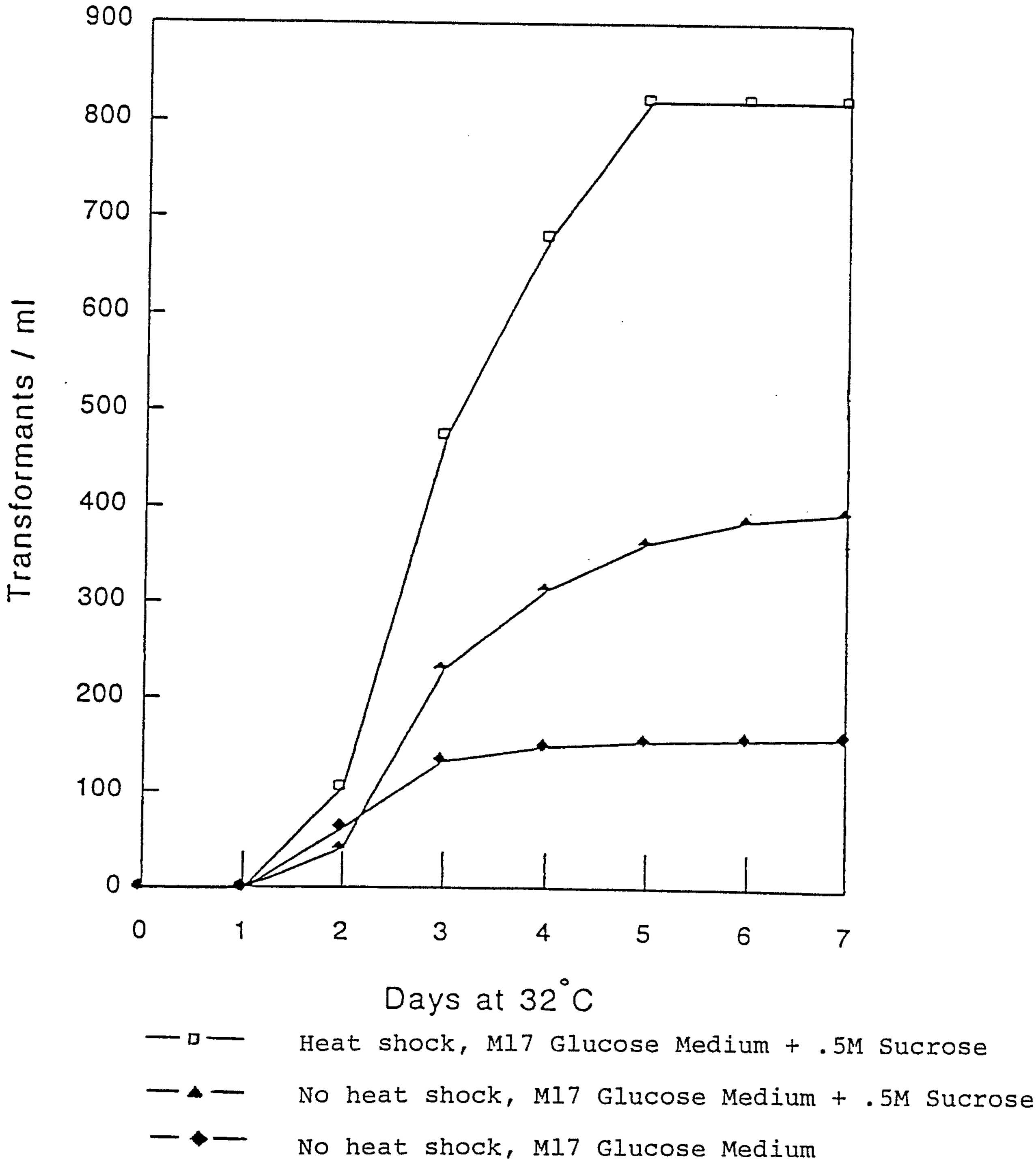
Transformants / ml
900
800
700
600
500
400
300
200
100
0
0
1
2
3
4
5
6
7
Days at 32°C
Heat shock, M17 Glucose Medium + .5M Sucrose
No heat shock, M17 Glucose Medium + .5M Sucrose
No heat shock, M17 Glucose Medium

FIG. 2

MS 1466

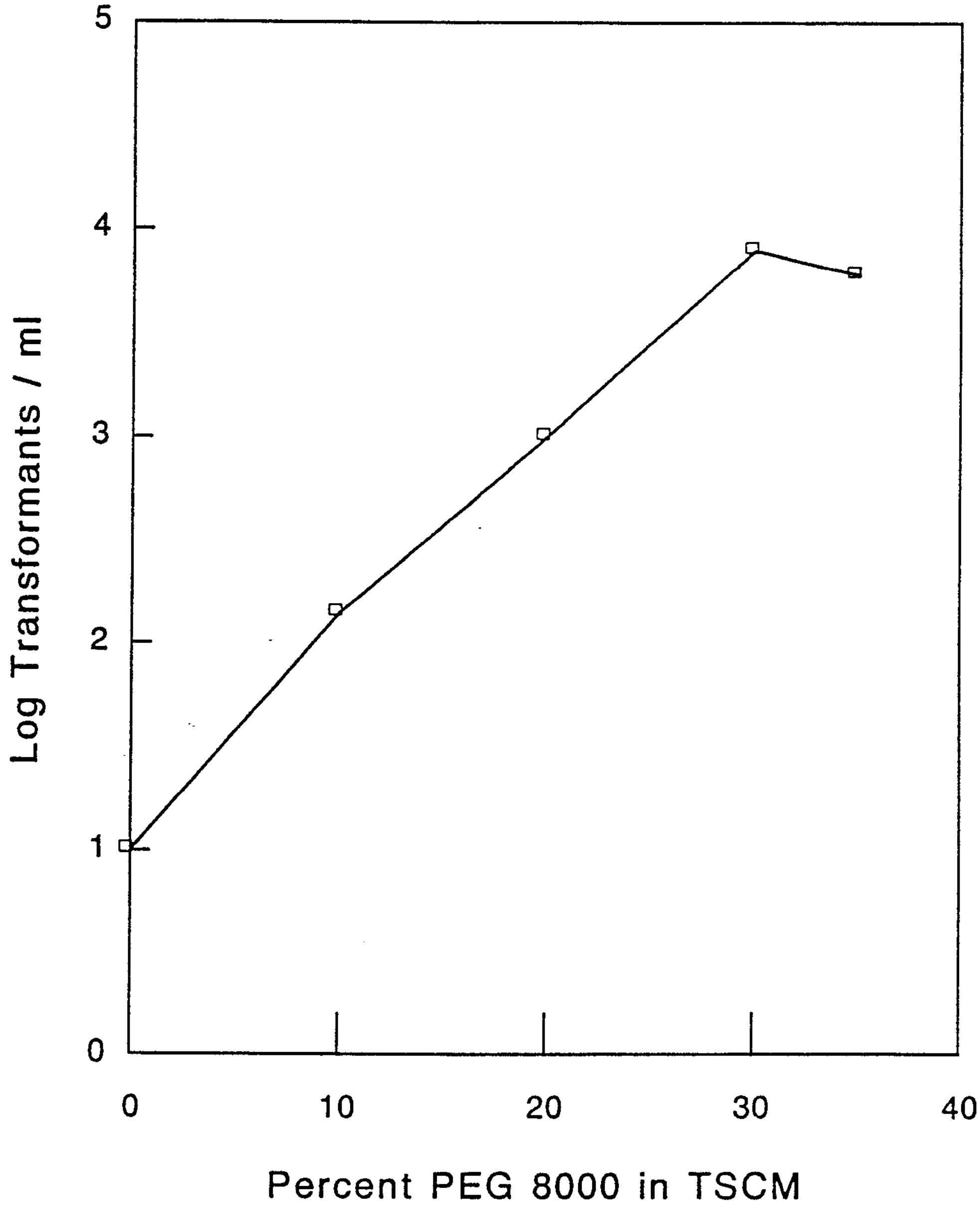

5
4
3
2
1
0
Log Transformants / ml
0
10
20
30
40
Percent PEG 8000 in TSCM


FIG. 3

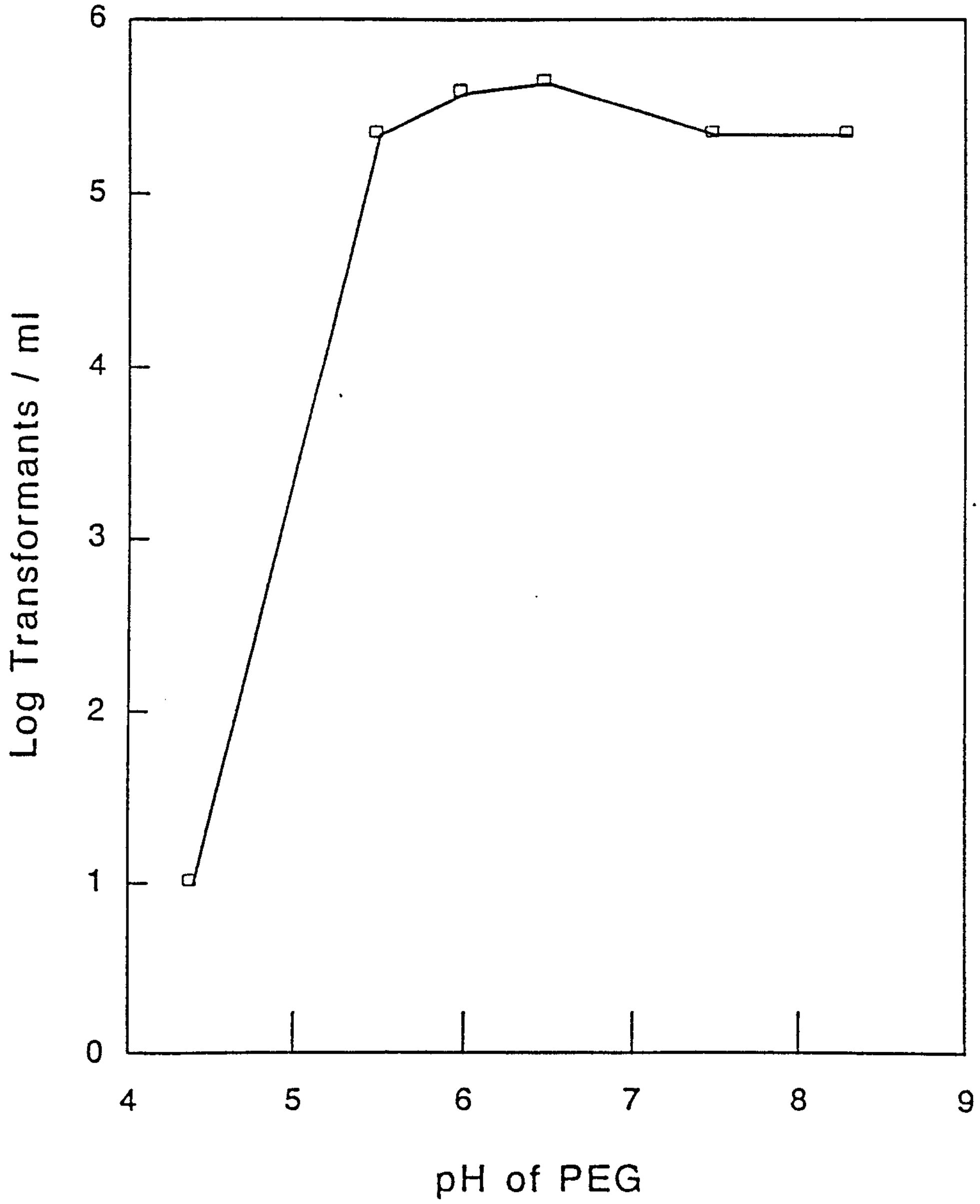


FIG. 4

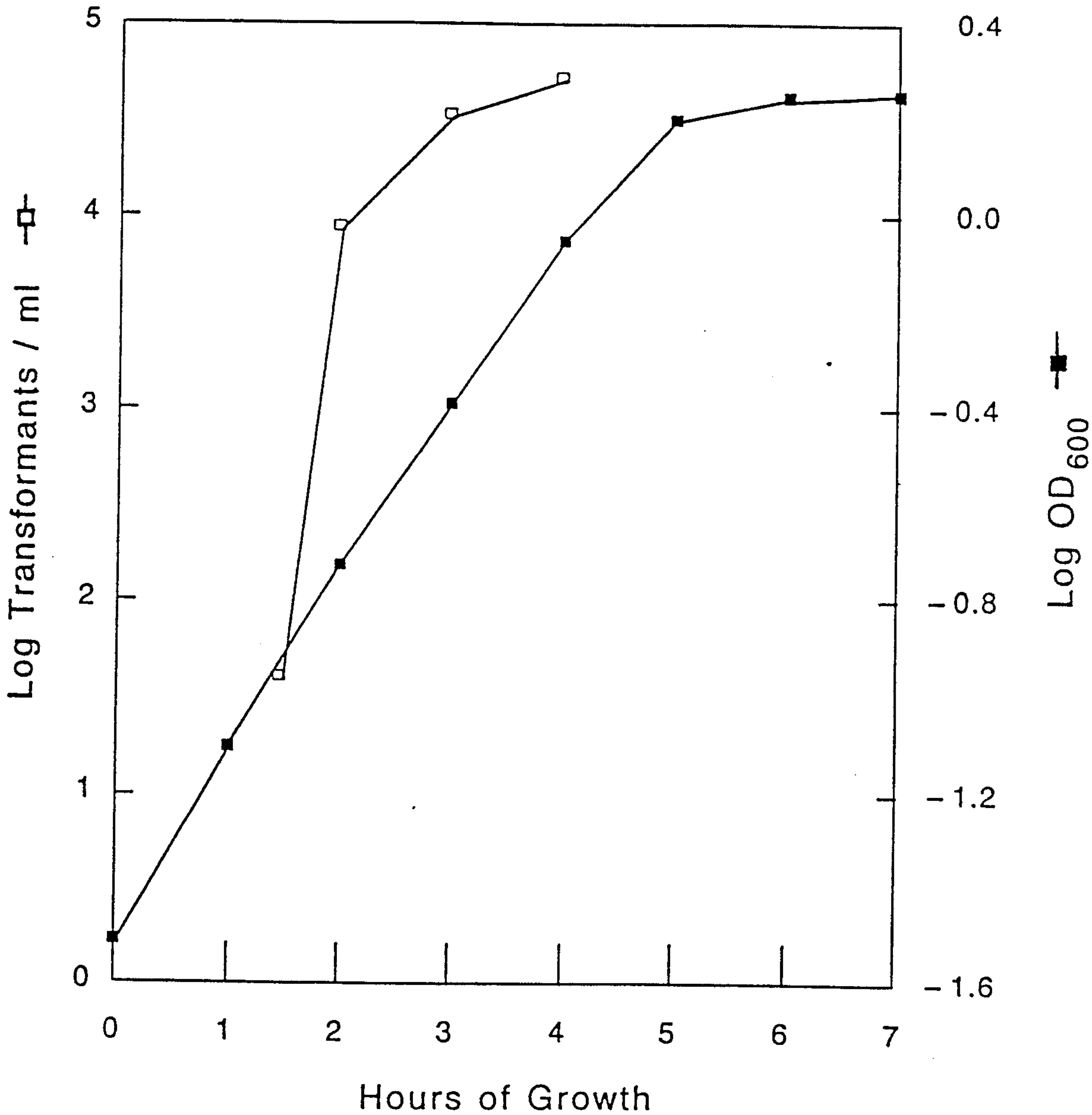
5
4
3
2
1
0
Log Transformants / ml
0.4
0.0
-0.4
-0.8
-1.2
-1.6
Log $OD_{600}$
0
1
2
3
4
5
6
7
Hours of Growth

FIG. 5

MS 1466

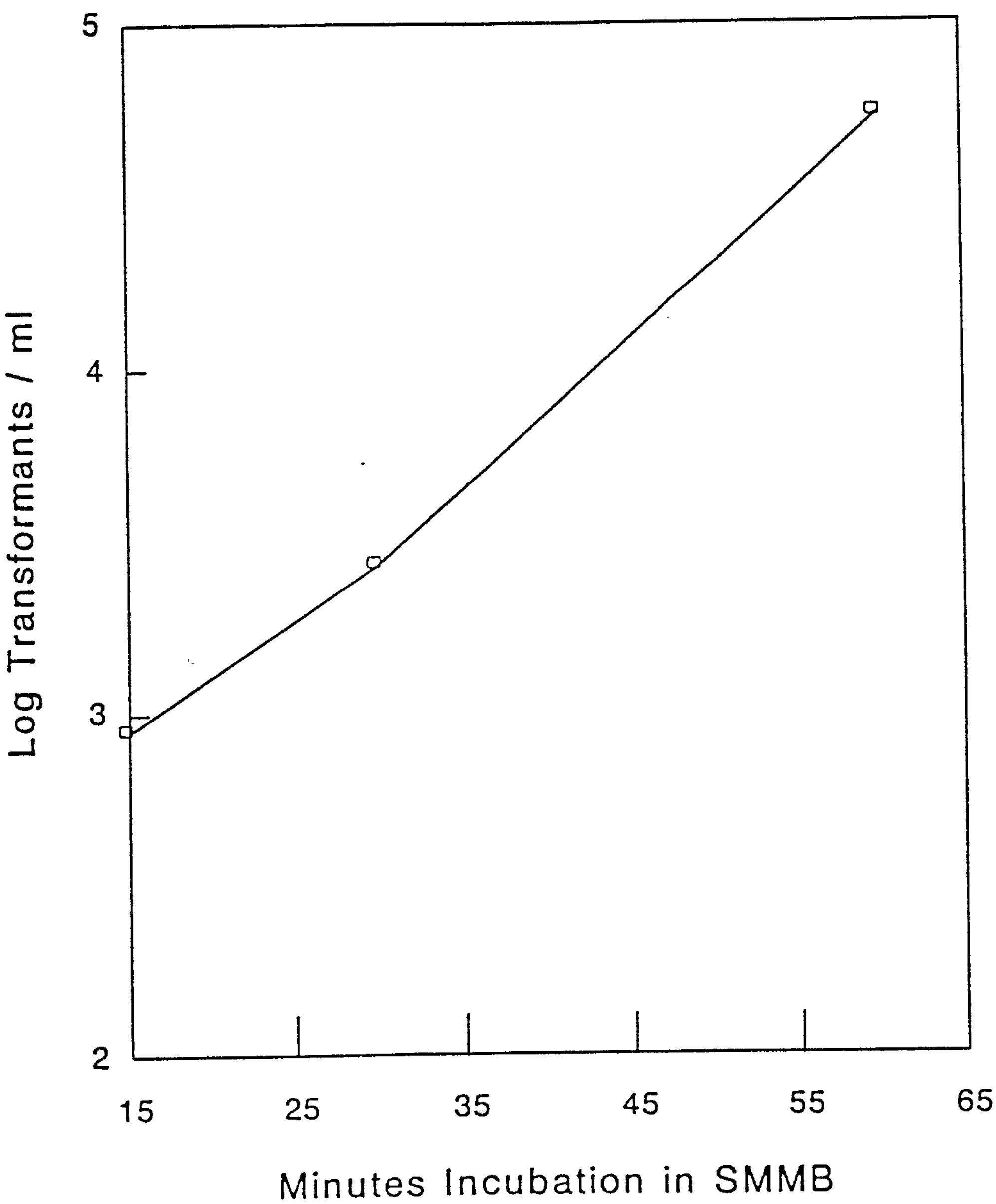
5
4
3
2
Log Transformants / ml
15
25
35
45
55
65
Minutes Incubation in SMMB

FIG. 6